# EUROPÄISCHE PATENTANMELDUNG

(11) **EP 2 319 848 A1**
(43) Veröffentlichungstag der Anmeldung: **11.05.2011**
(21) Anmeldenummer: 11000310.0
(22) Anmeldetag: 27.11.2003
(51) Int. Cl.: C07D 519/00, C07D 495/04, C08G 61/12

(54) **2,2'-Di (3,4-alkylendioxythiophen)-Derivate und deren Verwendung zur Herstellung von 2,2'-Poly-(3,4-alkylendioxythiophen)-Derivaten**

(30) Priorität: 10.12.2002 DE 10257539
(62) Teilanmeldung aus: 03027357.7
(71) Anmelder: H.C. Starck Clevios GmbH, 38642 Goslar (DE)
(72) Erfinder: Reuter, Knud, Dr., 47800 Krefeld (DE)
(74) Vertreter: Herzog, Martin

(57) **Zusammenfassung**

Die Erfindung betrifft ein Verfahren zur Herstellung von neutralen oder kationischen Verbindungen der allgemeinen Formel (IV), 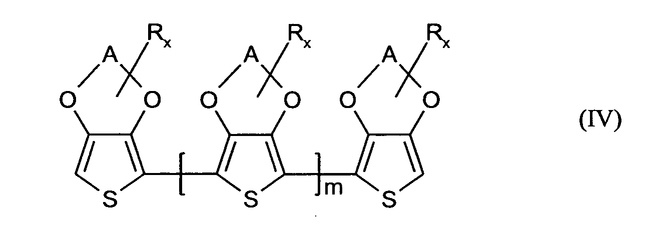 bei dem Verbindungen der allgemeinen Formel (I) 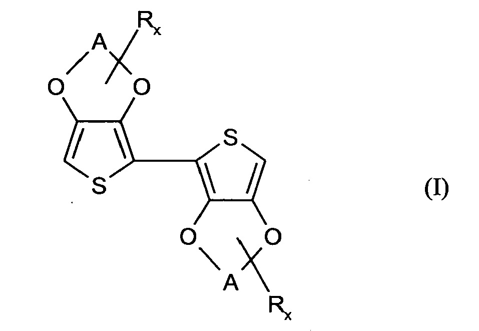 chemisch oder elektrochemisch oxidativ polymerisiert werden.

## Beschreibung

Die Erfindung betrifft ein Verfahren zur Herstellung von 2,2'-Di(alkylendioxythiophen)en (in der Literatur auch 2,2'-Bis(3,4-alkylendioxythiophen)e oder 3,4-(Alkylendioxy)-2,2'-bithiophene genannt), neuartige Verbindungen dieser Substanzklasse sowie deren Verwendung als wichtige Vorprodukte bzw. zur Herstellung wichtiger Vorprodukte für π-konjugierte Polymere.

Die Verbindungsklasse der π-konjugierten Polymere war in den letzten Jahrzehnten Gegenstand zahlreicher Veröffentlichungen. Sie werden auch als leitfähige Polymere oder als synthetische Metalle bezeichnet.

Wegen der erheblichen Delokalisierung der π-Elektronen entlang der Hauptkette zeigen diese Polymere interessante (nichtlineare) optische Eigenschaften, und nach Oxidation oder Reduktion stellen sie gute elektrische Leiter dar. Dadurch können diese Verbindungen in verschiedenen praktischen Anwendungsgebieten eingesetzt werden, wie z.B. in der Datenspeicherung, der optischen Signalverarbeitung, der Unterdrückung elektromagnetischer Störungen (EMI) und der Sonnenenergieumwandlung, sowie in wiederaufladbaren Batterien, Licht emittierenden Dioden (LED's), Feldeffekttransistoren, Leiterplatten, Sensoren, Kondensatoren und antistatischen Materialien.

Beispiele für bekannte π-konjugierte Polymere sind Polypyrrole, Polythiophene, Polyaniline, Polyacetylene, Polyphenylene und Poly(p-phenylen-vinylene).

Ein besonders wichtiges und technisch genutztes Polythiophen ist das Poly(3,4-ethylendioxythiophen), das in seiner kationischen Form sehr hohe Leitfähigkeiten aufweist. Weiterhin sind eine Reihe von Polymeren bekannt, die den 3,4-Ethylendioxythiophen-Baustein enthalten und wegen ihrer Elektrolumineszenz als Rohstoffe für Licht emittierende Dioden geeignet sind. Dabei ist es zur Einstellung der Emissions-Wellenlängen und der Lumineszenz-Intensität besonders wertvoll, auch auf 2,2'-Di(3,4-ethylendioxythiophen)-Bausteine zurückgreifen zu können. (Lit. z. B. A. Donat-Bouillud, I. Lévesque, Y. Tao, M. D'Iorio, S. Beaupré, P. Blondin, M. Ranger, J. Bouchard und M. Leclerc, Chem. Mater. 2000, 12, S. 1931-1936).

Problematisch für diesen Einsatz ist, dass die benötigten 2,2'-Di(3,4-ethylendioxythiophen)e bisher nur über aufwändige metallorganische Synthesen zugänglich sind, die - je nach Reaktionsbedingungen - zu geringen Ausbeuten und/oder zu nur mäßigen Reinheiten führen können.

Die 2,2'-Di(3,4-ethylendioxythiophen)e werden in der Literatur bisher in der Regel nach folgendem Verfahren (Ullmann-Kupplung) hergestellt:
3,4-Ethylendioxythiophen wird mittels n-Butyllithium bei -78°C in absolutem Tetrahydrofuran unter Schutzgas lithiiert und anschließend mit Kupfer-II-chlorid oxidativ zum 2,2'-Di(3,4-ethylendioxythiophen) gekuppelt.

Die im Folgenden aufgeführten Originalarbeiten illustrieren die Nachteile dieses Syntheseweges, insbesondere bezüglich der Reaktionsbedingungen, Ausbeuten und Reinheiten:
Aus G. A. Sotzing, J. R. Reynolds und P. J. Steel, Adv. Mater. 1997, 9(10), S. 795 - 798 geht hervor, dass nur ein unreines Produkt erhalten wurde (Schmp. 183 - 185°C). In A. Donat-Bouillud, I. Levesque, Y. Tao, M. D'Iorio, S. Beaupré, P. Blondin, M. Ranger, J. Bouchard und M. Leclerc, Chem. Mater. 2000, 12, S. 1931-1936 wird zwar nach gleichem Verfahren die Herstellung eines reineren Produktes beschrieben (Schmp. 203°C), jedoch beträgt die Ausbeute nur 27,7 % der Theorie. Durch eine Verlängerung der Reaktionszeit für die Ullmann-Kupplung wurde von A. K. Mohanakrishnan, A. Hucke, M. A. Lyon, M. V. Lakshmikantham und M. P. Cava (Tetrahedron 55 (1999), S. 11745 - 11754) eine Ausbeute von 84 % eines relativ reinen Produktes erhalten (Schmp. 203 - 204°C). Hierfür musste die Reaktionszeit jedoch versechsfacht werden.

Wesentliche Nachteile des in allen diesen Publikationen benutzten Verfahrens zur Herstellung von 2,2'-Di(3,4-ethylendioxythiophen) sind: 1) Für die Lithiierung muss bei tiefen Temperaturen (- 78°C) mit Hilfe einer externen Kältemischung, zum Beispiel aus Aceton/Trockeneis, gearbeitet werden. 2) n-Butyllithium als metallorganisches Reagens ist kostspielig und in der Handhabung luft- und feuchtigkeitsempfindlich sowie, insbesondere bei Hinzutritt von Feuchtigkeit, leicht entzündlich. 3) Die gesamte Reaktion muss wegen der Empfindlichkeit des Butyllithiums und des als Zwischenprodukt gebildeten lithiierten Thiophens unter Schutzgas (Stickstoff oder Argon) durchgeführt werden.

Die von S. S. Zhu und T. M. Swager in J. Am. Chem. Soc. 1997, 119, S. 12568 - 12577 angegebene Variante dieses Verfahrens (Lithiierung bei -10°C unter Zusatz von Tetramethylethylendiamin und oxidative Kupplung mittels Eisen-IIIacetylacetonat unter Rückfluss in THF) ergibt keine Vereinfachungen, da Kältemischungen und Schutzgastechnik ebenfalls erforderlich sind. Die tatsächliche Ausbeute beträgt 50 % der Theorie (0,99 g von maximal möglichen 1,97 g); die angegebenen 99 % beruhen auf einem Druckfehler.

Ein weiterer wesentlicher Nachteil der zitierten metallorganischen Verfahrensweisen ist deren eingeschränkte Anwendbarkeit. An der Ethylen-Brücke substituierte 2,2'-Di(3,4-ethylendioxythiophen)e sind daher bisher nicht beschrieben worden. Sie wären nach der Methode der Lithiierung mit anschließender Ullmann-Reaktion nicht auf gezieltem Weg zugänglich, da bei zahlreichen denkbaren Substituenten an der Ethylen-Brücke - z. B. Hydroxyalkylgruppen, Carbonyl- oder Heterocarbonylgruppen, Doppelbindungen etc. - eine Lithiierung dieser Substituenten als Nebenoder Hauptreaktion einträte. Auch 2,2'-Di(3,4-alkylendioxythiophen)e mit anderen Alkylenbrücken als der Ethylenbrücke wurden bisher in der Literatur nicht beschrieben.

Somit besteht Bedarf nach einer neuen Methode zur Herstellung bekannter sowie neuartiger 2,2'-Di(3,4-alkylendioxythiophen)e, bei der
- auf den Einsatz von niedrigen Temperaturen, d. h. Kältemischungen oder ähnlichem zur externen Kühlung, verzichtet werden kann
- eine Durchführung ohne Schutzgastechnik an Luft möglich ist
- eine breite Anwendbarkeit, beispielsweise für die Herstellung an der Ethylen-Brücke funktionell substituierter 2,2'-Di(3,4-ethylendioxythiophen)e oder sonstiger gegebenenfalls substituierter 2,2'-Di(3,4-alkylendioxythiophen)e, gegeben ist.

Diese Aufgabe wird erfindungsgemäß dadurch gelöst, dass auf einfache Weise 2,2'-Di(3,4-alkylendioxythiophen)e, im Folgenden auch als. Verbindungen der Formel (I) bezeichnet, hergestellt werden können, indem Verbindungen der allgemeinen Formel (II), 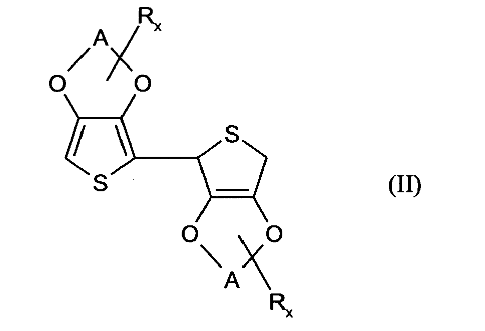 worin
- A: für einen gegebenenfalls substituierten C₂-C₄-Alkylenrest steht,
- R: für einen oder mehrere, gleiche oder verschiedene lineare(n) oder verzweigte(n), gegebenenfalls substituierte(n) C₁-C₁₈-Alkylrest(e), gegebenenfalls substituierte(n) C₅-C₁₂-Cycloalkylrest(e), gegebenenfalls substituierte(n) C₆-C₁₄Arylrest(e), gegebenenfalls substituierte(n) C₁-C₄-Hydroxyalkylrest(e) oder einen oder mehrere Hydroxylrest(e) steht,
- x: für eine ganze Zahl von 0 bis 8 steht,
mit einem Dehydrierungsmittel umgesetzt werden.

Gegenstand der vorliegenden Erfindung ist daher ein Verfahren zur Herstellung von Verbindungen der allgemeinen Formel (I), 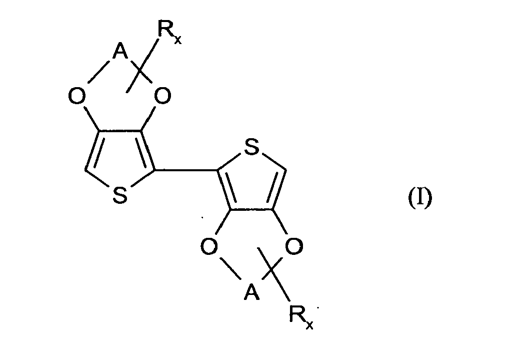 worin
- A: für einen gegebenenfalls substituierten C₂-C₄-Alkylenrest, bevorzugt für einen gegebenenfalls substituierten Ethylenrest steht,
- R: für einen oder mehrere, gleiche oder verschiedene lineare(n) oder verzweigte(n), gegebenenfalls substituierte(n) C₁-C₁₈-Alkylrest(e), gegebenenfalls substituierte(n) C₅-C₁₂-Cycloalkylrest(e), gegebenenfalls substituierte(n) C₆-C₁₄-Arylrest(e), gegebenenfalls substituierte(n) C₁-C₄-Hydroxyalkylrest(e) oder einen oder mehrere Hydroxylrest(e), bevorzugt für Methyl, Ethyl, n-Propyl, n-Butyl, n-Hexyl, n-Octyl, n-Decyl, n-Dodecyl, n-Tetradecyl oder Hydroxymethyl steht,
- x: für eine ganze Zahl von 0 bis 8, bevorzugt für eine ganze Zahl von 0 bis 4, besonders bevorzugt für 0 oder 1 steht,
**dadurch gekennzeichnet, dass** Verbindungen der allgemeinen Formel (II), 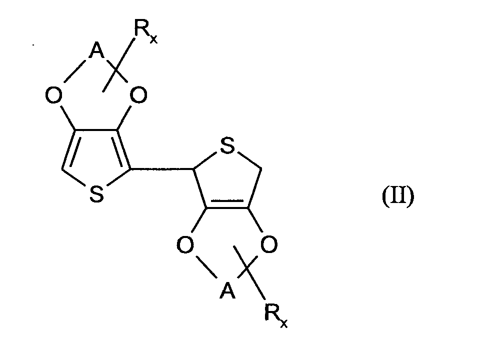 worin A, R und x die für die allgemeine Formel (I) genannte Bedeutung haben,
mit einem Dehydrierungsmittel umgesetzt werden.

Eine bevorzugte Ausführungsform des erfindungsgemäßen Verfahrens ist ein solches zur Herstellung der Verbindung der Formel (I-a) (auch als 2,2'-Di(3,4-ethylendioxythiophen) bezeichnet) 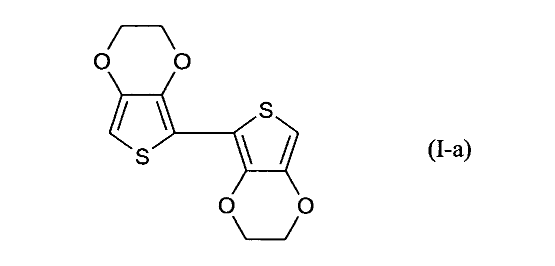 **dadurch gekennzeichnet, dass** Verbindungen der Formel (II-a) 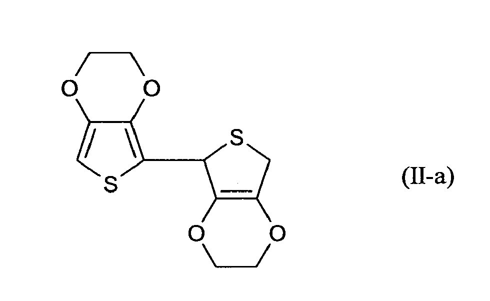 mit einem Dehydrierungsmittel umgesetzt werden.

Die Verbindungen der Formel (II) können je nach Wahl von A und x verschiedene Stereoisomeren, d.h. Enantiomeren oder Diastereoisomeren, umfassen. Im Rahmen der Erfindung sind unter Verbindungen der Formel (II) entweder die reinen Enantiomeren oder Diastereoisomeren oder Mischungen aus diesen in einem beliebigen Mischungsverhältnis zu verstehen. Beispielsweise können Verbindungen der Formel (II-a) die beiden Enantiomeren (II-a-S) oder (II-a-R) 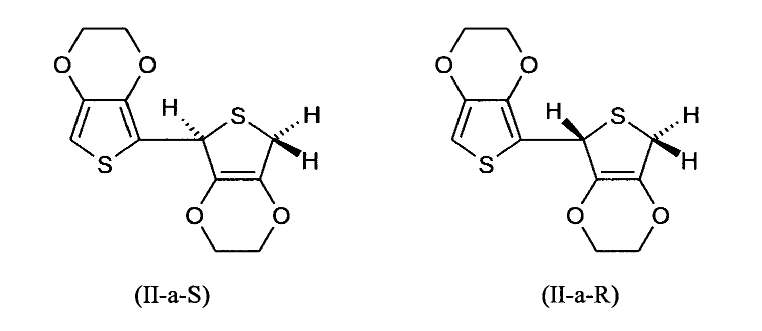 in reiner Form oder Mischungen aus diesen in beliebigen Mischungsverhältnissen von (II-a-S) zu (II-a-R) sein.

Die Verbindungen der Formel (II) können nach einem Verfahren hergestellt werden, welches in der noch nicht veröffentlichten deutschen Patentanmeldung DE 10 229 218 beschrieben ist. Dazu werden Verbindungen der Formel (VI) 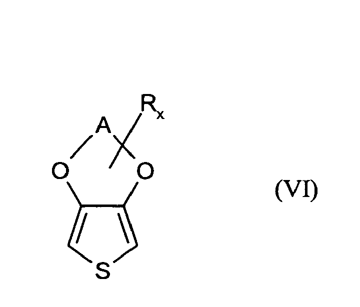 worin A, R und x die für die allgemeine Formel (I) genannte Bedeutung haben, in Gegenwart von Lewis-Säuren, bevorzugt nicht-oxidierenden Lewis-Säuren wie beispielsweise Bortrifluorid (z.B. in Form des Etherats oder eines anderen BF₃-Komplexes, z.B. mit Tetrahydrofuran), Antimonpentachlorid, Aluminiumtrichlorid, Titantetrachlorid, Zinntetrachlorid und Zinkchlorid, oder in Gegenwart von Protonensäuren, bevorzugt ausgewählt aus der Gruppe der Sulfon- oder Carbonsäuren - wie beispielsweise p-Toluolsulfonsäure, Methansulfonsäure, Campher-10-sulfonsäure oder Trifluoressigsäure - oder Supersäuren, als Katalysator miteinander umgesetzt werden. Die Verbindungen der allgemeinen Formel (VI) werden beispielsweise in einem organischen Lösungsmittel, z.B. halogenierten aliphatischen Kohlenwasserstoffen, wie Methylenchlorid oder Chloroform, Alkoholen, wie Methanol oder Ethanol, oder Aromaten, wie Toluol oder Xylol, bei Temperaturen von 0°C bis 40°C gegebenenfalls unter Schutzgas mit dem Katalysator umgesetzt. Dabei werden bevorzugt 0,01 bis 50 Gew.-% Lewissäure oder 0,5 bis 80 Gew.-% Protonensäure als Katalysator eingesetzt (Gew.% bezogen auf das zu dimerisierende Thiophenmonomer). Die Umsetzung kann mehrere Stunden durchgeführt oder ¹H-NMR-spektroskopisch verfolgt und zum geeigneten Zeitpunkt durch Zugabe von Basen (z.B. wässrige Na₂CO₃-Lösung) oder Alkoholen (z.B. Ethanol) abgebrochen werden. Die erhaltenen Verbindungen der allgemeinen Formel (II) können säulenchromatographisch aufgereinigt werden.

C₂-C₄-Alkylenreste A sind im Rahmen der Erfindung Ethylen, n-Propylen oder n-Butylen. Lineare oder verzweigte C₁-C₁₈-Alkylreste sind im Rahmen der Erfindung beispielsweise Methyl, Ethyl, n- oder iso-Propyl, n-, iso-, sec- oder tert-Butyl, n-Pentyl, 1-Methylbutyl, 2-Methylbutyl, 3-Methylbutyl, 1-Ethylpropyl, 1,1-Dimethylpropyl, 1,2-Dimethylpropyl, 2,2-Dimethylpropyl, n-Hexyl, n-Heptyl, n-Octyl, 2-Ethylhexyl, n-Nonyl, n-Decyl, n-Undecyl, n-Dodecyl, n-Tridecyl, n-Tetradecyl, n-Hexadecyl oder n-Octadecyl, C₅-C₁₂-Cycloalkylreste sind beispielsweise Cyclopentyl, Cyclohexyl, Cycloheptyl, Cyclooctyl, Cyclononyl oder Cyclodecyl, C₆-C₁₄-Arylreste sind beispielsweise Phenyl, o-, m-, p-Tolyl, Benzyl, 2,3-, 2,4-, 2,5-, 2,6-, 3,4-, 3,5-Xylyl, Mesityl oder Naphthyl, und C₁-C₄-Hydroxyalkylreste sind beispielsweise Hydroxymethyl, 1-Hydroxyethyl, 2-Hydroxyethyl, 1-Hydroxypropyl, 2-Hydroxypropyl, 3-Hydroxypropyl, 1-Hydroxybutyl, 2-Hydroxybutyl, 3-Hydroxybutyl, 4-Hydroxybutyl. Die vorangehende Aufzählung dient der beispielhaften Erläuterung der Erfindung und ist nicht als abschließend zu betrachten.

Als Substituenten der oben genannten Reste kommen zahlreiche organische Gruppen in Frage, beispielsweise Halogen-, insbesondere Fluor, Chlor oder Brom, sowie Ether-, Thioether-, Disulfid-, Sulfoxid-, Sulfon-, Aldehyd-, Keto-, Carbonsäureester-, Sulfonsäureester-, Carbonat-, Cyano-, Alkylsilan- und Alkoxysilangruppen und/oder Carboxylamidgruppen.

Als Dehydrierungsmittel im erfindungsgemäßen Verfahren eignen sich solche, die zur Dehydrierung von 2,3-Dihydro-, 2,5-Dihydro- oder Tetrahydrothiophenen sowie deren Derivaten geeignet sind. Bevorzugt sind dies solche, die eine Dehydrierung der Verbindungen der allgemeinen Formel (II) bewirken, jedoch keine anschließende oxidative Polymerisation der erhaltenen Verbindungen der allgemeinen Formel (I) unterstützen. Durch die Dehydrierung findet eine Aromatisierung der Dihydrothiophene bzw. Verbindungen der allgemeinen Formel (II) statt. Die Dehydrierung kann im Sinne der Erfindung auf unterschiedlichen Wegen erfolgen:
- durch Thiolierung und Eliminierung von Schwefelwasserstoff
- durch Hydridionentransfer
- durch Halogenierung mittels Halogenierungsreagenzien und Eliminierung von Halogenwasserstoff
- durch S-Oxidation mittels geeigneter Oxidationsmittel und Dehydratisierung.

Bevorzugt eingesetzte Dehydrierungsmittel im erfindungsgemäßen Verfahren sind Chinone, Schwefel, Brom, N-Brom- oder N-Chlorsuccinimid, Sulfurylchlorid, Wasserstoffperoxid oder hypervalente Iodverbindungen wie Iodosobenzol. Bei der Dehydrierung mit Chinonen findet eine direkte Hydrierung des eingesetzten Dehydrierungsmittels statt (Hydridionentransfer), bei Einsatz von beispielsweise Brom, N-Brom- oder N-Chlorsuccinimid oder Sulfurylchlorid findet eine Halogenierung und nachfolgende Eliminierung von Halogenwasserstoff statt, Wasserstoffperoxid oder Iodosobenzol beispielsweise bewirken eine S-Oxidation gefolgt von einer Dehydratisierung und Schwefel bewirkt eine Thiolierung gefolgt von einer Eliminierung von Schwefelwasserstoff. Derartige Reaktionen sind beschrieben in Houben-Weyl, Methoden der Organischen Chemie (4. Aufl., Band E 6a (Hetarene I, Teil 1), Hrsg. R. P. Kreher, Georg Thieme-Verl., Stuttgart-New York 1994, im Kapitel "Thiophene" (Autor: W.-D. Rudorf) auf den Seiten 370 bis 388 in den Abschnitten 3. 3. bis 3. 8.).

Besonders bevorzugte Dehydrierungsmittel für den Einsatz im erfindungsgemäßen Verfahren sind Chinone, darunter ganz besonders bevorzugt 2,3,5,6-Tetrachlor-1,4-benzochinon (Chloranil) oder 2,3-Dichlor-5,6-dicyan-1,4-benzochinon.

Das erfindungsgemäße Verfahren wird prinzipiell so durchgeführt, dass die Verbindungen der allgemeinen Formel (II) mit mindestens äquimolaren oder überschüssigen Mengen an Dehydrierungsmittel bei geeigneter Temperatur und gegebenenfalls in einem organischen Lösemittel für 0,5 bis 48, bevorzugt 2 bis 24 h umgesetzt werden. Bevorzugt werden auf 1 Mol der Verbindung der allgemeinen Formel (II) 1 bis 2 Mol, besonders bevorzugt 1 bis 1,2 Mol an Dehydrierungsmittel eingesetzt, wobei in vielen Fällen ein genau äquimolares Verhältnis zwischen der Verbindung der allgemeinen Formel (II) und Dehydrierungsmittel ausreichend ist. Gegebenenfalls ausgefallener Feststoff wird abfiltriert und durch Aufschlämmen und anschließendes Filtrieren von unlöslichen Bestandteilen befreit. Zur Aufschlämmung eignet sich beispielsweise ein Gemisch aus organischem Lösemittel und wässriger Alkalilauge, wie z.B. 4%iger wässriger KOH-Lösung. Nach basischem Waschen und anschließendem Neutralwaschen mit Wasser sowohl dieser erhaltenen Mutterlauge als auch der Mutterlauge, die nach Filtration der Reaktionsmischung erhalten wurde, werden die Mutterlaugen eingeengt und im Vakuum getrocknet. Zum basischen Waschen eignen sich ebenfalls wässriger Alkalilaugen, wie z.B. 4%ige wässrige KOH-Lösung. Das erhaltene Rohprodukt kann nach gängigen Methoden, wie beispielsweise Säulenchromatographie oder durch Umkristallisation, aufgereinigt werden; weiterhin eignet sich hierzu auch die Entfernung stark färbender Verunreinigungen mit Bleicherde (Montmorillonit), z. B. Tonsil^{®}.

Geeignete Reaktionstemperaturen für die Durchführung des erfindungsgemäßen Verfahrens sind z. B. 20 bis 200°C, bevorzugt 100 bis 170°C, wobei es vorteilhaft sein kann, unter Rückfluss des verwendeten Lösemittels zu arbeiten. Grundsätzlich sind zudem auch niedrigere Temperaturen von 20°C bis hin zu -78°C geeignet, jedoch aufgrund der aufwändigen externen Kühlung bzw. Verwendung von Kältemischungen von untergeordneter Bedeutung. Geeignete Lösemittel sind z. B. gegebenenfalls halogenierte aromatische Kohlenwasserstoffe, wie Toluol, Xylol, Chlorbenzol oder o-Dichlorbenzol. Es können allerdings auch weitere unter den Reaktionsbedingungen inerte, vorzugsweise organische, Lösemittel gegebenenfalls im Gemisch mit Wasser verwendet werden.

Nach dem erfindungsgemäßen Verfahren sind erstmals Verbindungen der allgemeinen Formel (I) zugänglich, welche an der Ethylenbrücke substituiert sein können und solche, die sogar andere gegebenenfalls substituierte Alkylenbrücken als die Ethylenbrücke tragen. Derartige Verbindungen der Formel (I) sind bisher in der Literatur nicht beschrieben.

Gegenstand der vorliegenden Erfindung sind daher ebenfalls Verbindungen der allgemeinen Formel (I) 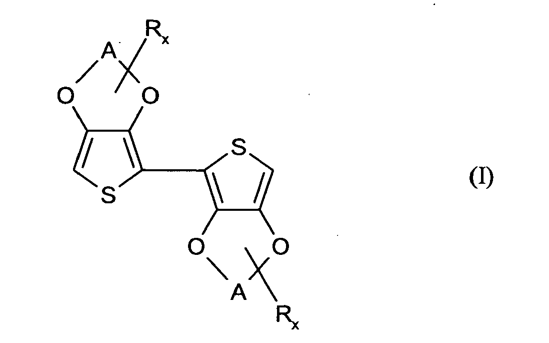 worin
A, R und x die oben genannte Bedeutung haben, ausgenommen die Verbindung der Formel (I-a)

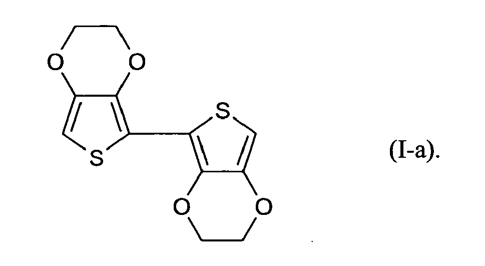

Verbindungen der allgemeinen Formel (I), worin beispielsweise A ein Ethylenrest und x = 1 ist, können im Sinne der Erfindung nicht nur eines der drei Konstitutionsisomeren in reiner Form 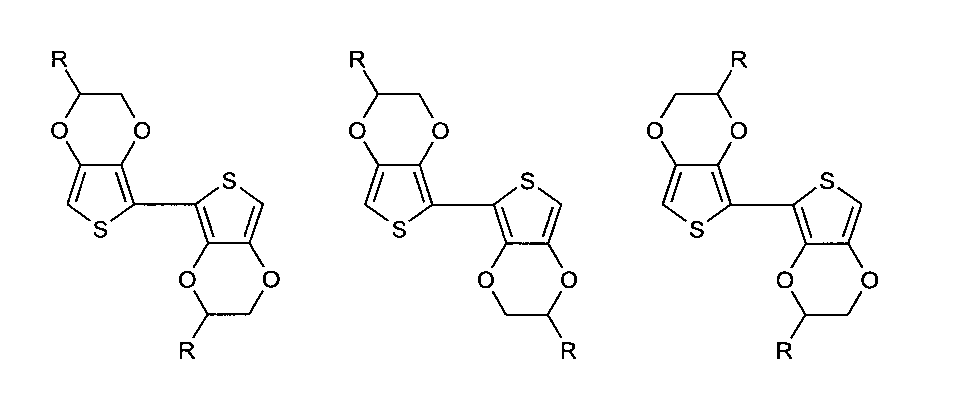 sondern auch Mischungen aus diesen in beliebigen Mischungsverhältnissen sein. Zudem können die verschiedenen Konstitutionsisomeren in unterschiedlichen stereoisomeren Formen auftreten, welche ebenfalls in reiner Form oder als Mischungen in beliebigen Mischungsverhältnissen im Sinne der Erfindung unter Verbindungen der allgemeinen Formel (I) verstanden werden. Dies gilt ebenfalls für Verbindungen der allgemeinen Formel (I), in denen A und/oder x eine andere Bedeutung als Ethylen (A) bzw. 1 (x) haben, sofern verschiedene Isomere strukturell möglich sind.

Die Verbindungen der allgemeinen Formel (I) eignen sich sowohl als Vorprodukte als auch zur Herstellung von wertvollen Vorprodukten bei der Herstellung π-konjugierter, elektrisch leitfähiger oder halbleitender Polymere oder sonstiger elektrisch leitfähiger oder halbleitender Verbindungen.

Weiterhin Gegenstand der vorliegenden Erfindung ist daher die Verwendung der Verbindungen der allgemeinen Formel (I) als Vorprodukte oder zur Herstellung von Vorprodukten für die Herstellung elektrisch leitfähiger oder halbleitender Verbindungen und/oder Polymere. Unter Polymeren sind in diesem Zusammenhang neben Homopolymeren auch π-konjugierte, elektrisch leitfähige oder halbleitende Copolymere umfasst, welche neben Monomereinheiten der allgemeinen Formel (V) 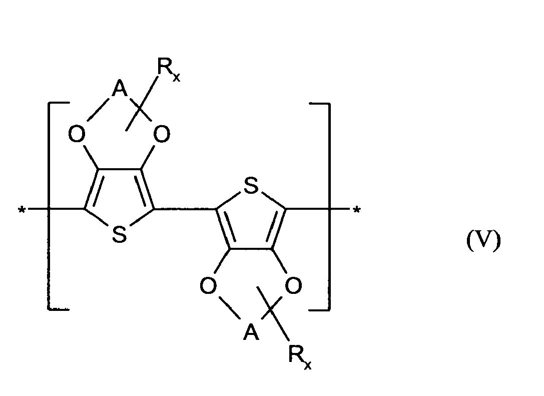 weitere geeignete Monomereinheiten, wie beispielsweise gegebenenfalls substituierte Fluoren-, Thiophen-, Furan-, Pyrrol-, Anilin-, Pyridin-, Carbazol- oder Phenyleneinheiten enthalten.

Unter elektrisch leitfähigen oder halbleitenden Verbindungen sind in diesem Zusammenhang beispielsweise gegebenenfalls substituierte π-konjugierte Oligomere enthaltend mindestens vier gleiche oder unterschiedliche (Hetero)arylen-Einheiten, wie beispielsweise Fluoren-, Thiophen-, Furan-, Pyrrol-, Anilin-, Pyridin-, Carbazol- oder Phenyleneinheiten, wobei mindestens eine Monomereinheit der allgemeinen Formel (V) (entsprechend zwei (Hetero)arylen-Einheiten) enthalten ist, zu verstehen. In Halbleiterfachkreisen sind solche Verbindungen auch als ,,small molecules" geläufig.

Bevorzugt Gegenstand der vorliegenden Erfindung ist die Verwendung der erfindungsgemäß hergestellten bzw. erfindungsgemäßen Verbindungen der allgemeinen Formel (I) zur Herstellung elektrisch leitfähiger oder halbleitender Poly(3,4-ethylendioxythiophene) oder zur Herstellung von 5,5'-Dihalogen-2,2'-di-(3,4-alkylendioxythiophen)en.

Die erfindungsgemäß hergestellten Verbindungen der allgemeinen Formel (I) lassen sich beispielsweise durch oxidative Polymerisation, z.B. unter Verwendung von Eisen(III)-Verbindungen als Oxidationsmittel, zu den entsprechenden Poly(3,4-ethylendioxythiophenen) umsetzen, die als leitfähige oder halbleitende Polymere vielfältige Anwendung finden.

Weiterhin Gegenstand der vorliegenden Erfindung ist somit ein Verfahren zur Herstellung von neutralen oder kationischen Verbindungen der allgemeinen Formel (IV), 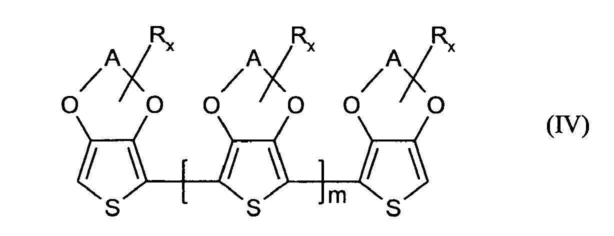 worin
A, R und x oben genannte Bedeutung haben,
m für eine ganze gerade Zahl von 2 bis 200 steht und
wobei die Verbindungen der allgemeinen Formel (IV) für den Fall, dass sie kationisch sind eine bis maximal m+2 positive Ladung(en) tragen,
**dadurch gekennzeichnet, dass** Verbindungen der allgemeinen Formel (I) 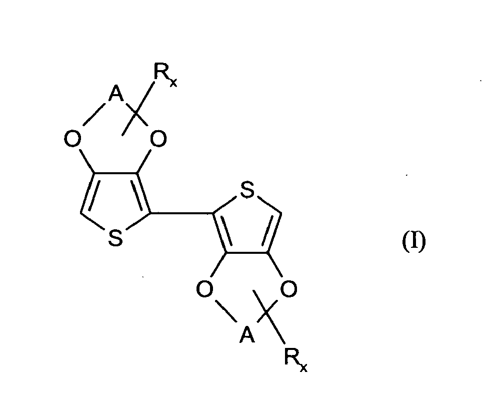 worin
A, R und x die oben genannte Bedeutung haben,
chemisch oder elektrochemisch oxidativ polymerisiert werden.

Die Polythiophene der allgemeinen Formel (IV) können neutral oder kationisch sein. Für den Fall, dass es sich um kationische Polythiophene handelt, werden die positiven Ladungen der Polythiophen-Polykationen nicht in der Formel (IV) dargestellt, da ihre genaue Zahl und ihre Position nicht einwandfrei feststellbar sind. Die Anzahl der positiven Ladungen beträgt jedoch mindestens 1 und höchstens m + 2.

Zur Kompensation der positiven Ladung enthält die kationische Form der Polythiophene Anionen, vorzugsweise Polyanionen, als Gegenionen.

Als Polyanionen dienen vorzugsweise die Anionen von polymeren Carbonsäuren, wie Polyacrylsäuren, Polymethacrylsäure oder Polymaleinsäuren und polymeren Sulfonsäuren, wie Polystyrolsulfonsäuren und Polyvinylsulfonsäuren. Diese Polycarbon- und -sulfonsäuren können auch Copolymere von Vinylcarbon- und Vinylsulfonsäuren mit anderen polymerisierbaren Monomeren, wie Acrylsäureestern und Styrol, sein.

Besonders bevorzugt ist das Anion der Polystyrolsulfonsäure als Gegenion.

Das Molekulargewicht der die Polyanionen liefernden Polysäuren beträgt vorzugsweise 1 000 bis 2 000 000, besonders bevorzugt 2 000 bis 500 000. Die Polysäuren oder ihre Alkalisalze sind im Handel erhältlich, z.B. Polystyrolsulfonsäuren und Polyacrylsäuren.

Die erfindungsgemäßen Verbindungen der allgemeinen Formel (I) können chemisch oder elektrochemisch oxidativ zu Polythiophenen der allgemeinen Formel (IV) umgesetzt werden. Das Verfahren sowohl der oxidativen chemischen als auch elektrochemischen Polymerisation von Polythiophenen bzw. 3,4-Alkylendioxythiophenderivaten ist dem Fachmann bekannt und in der Literatur ausführlich beschrieben (L. Groenendaal, F. Jonas, D. Freitag, H. Pielartzik und J. R. Reynolds, Adv. Mater. 2000, 12(7), S. 481 - 494). Als chemische Oxidationsmittel sind die aus dem Stand der Technik zur Polythiophenherstellung bekannten geeignet, beispielsweise Eisen-III-Verbindungen, wie FeCl₃ oder Eisen-III-tosylat, Kaliumpermanganat, Braunstein (MnO₂), Kalium(di)chromat oder Peroxodisulfate (Persulfate), wie Na₂S₂O₈ oder K₂S₂O₈, sowie H₂O₂.

Ferner können die erfindungsgemäß hergestellten Verbindungen bzw. erfindungsgemäßen Verbindungen durch Halogenierung, bevorzugt Chlorierung oder Bromierung, zu Verbindungen der allgemeinen Formel (III) (auch als 5,5'-Dihalogen-2,2'-di-(3,4-alkylendioxythiophene) bezeichnet), welche wertvolle Vorprodukte bei der übergangsmetallkatalysierten Herstellung π-konjugierter Polymere sind, umgesetzt werden. Das prinzipielle Verfahren ist dem Fachmann bekannt und beispielsweise in A. Donat-Bouillud, I. Lévesque, Y. Tao, M. D'Iorio, S. Beaupré, P. Blondin, M. Ranger, J. Bouchard und M. Leclerc, Chem. Mater. 2000, 12, S. 1931-1936 beschrieben.

Weiterhin Gegenstand der vorliegenden Erfindung ist somit ein Verfahren zur Herstellung von Verbindungen der allgemeinen Formel (III), 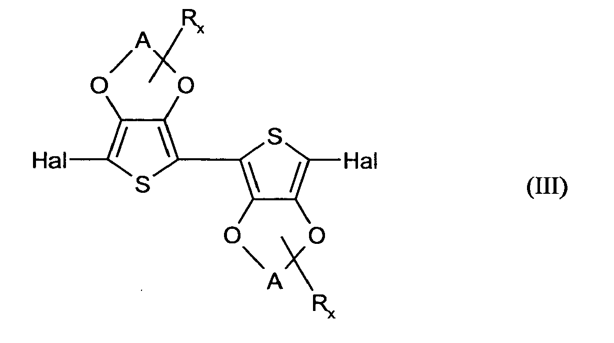 worin
A, R und x die oben genannte Bedeutung haben und
Hal für Cl oder Br steht,
ausgenommen die Verbindung der Formel (III-a) 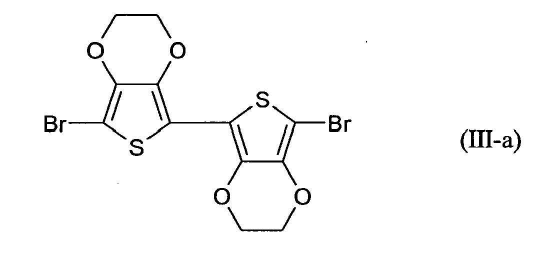 **dadurch gekennzeichnet, dass** Verbindungen der allgemeinen Formel (I) 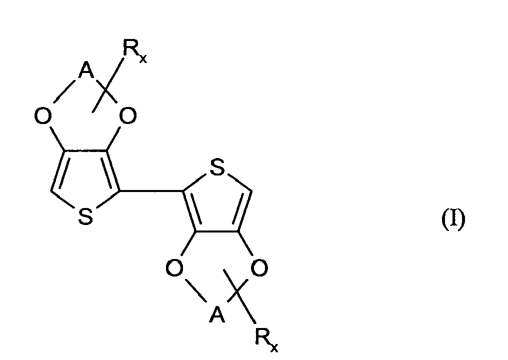 worin
A, R und x die oben genannte Bedeutung haben,
halogeniert werden.

### Beispiele

### Allgemeine Vorschrift zur Synthese der Verbindungen der allgemeinen Formel (II):

Beispielhaft kann die Synthese der Verbindungen der allgemeinen Formel (II) so erfolgen, dass eine Lösung des 3,4-Alkylendioxythiophens in Methylenchlorid mit 0,5 bis 80 Gew-% einer Protonensäure, z.B. p-Toluolsulfonsäure oder Trifluoressigsäure, oder 0,01 bis 50 Gew.-% einer Lewissäure, z.B. Bortrifluorid in Form des Etherats oder eines anderen BF₃-Komplexes, als Katalysator bei Temperaturen von 0°C bis 40°C gegebenenfalls unter N₂-Atmosphäre umgesetzt wird. Die Umsetzung kann mehrere Stunden durchgeführt oder ¹H-NMR-spektroskopisch verfolgt und zum geeigneten Zeitpunkt durch Zugabe von Basen (z.B. wässrige Na₂CO₃-Lösung) oder Alkoholen (z.B. Ethanol) abgebrochen werden. Die erhaltenen Verbindungen der allgemeinen Formel (II) können anschließend säulenchromatographisch, z.B. an Silicagel mit Methylenchlorid als Laufinittel, aufgereinigt werden.

### Herstellung von 2,2',3,3',5,7-Hexahydro-5,5'-bithieno[3,4-b][1,4]dioxin (II-a) (EDT-Dimer) durch Trifluoressigsäure-Katalyse

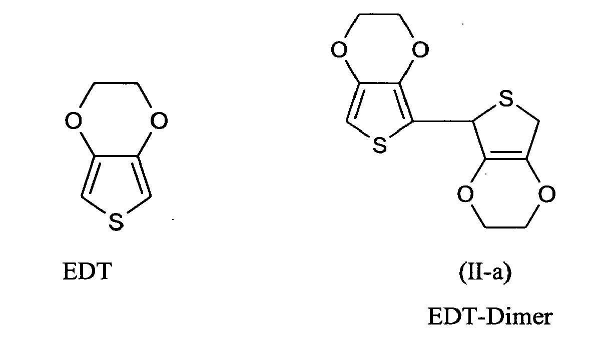

Wie in der allgemeinen Vorschrift beschrieben, wurden zu einer Lösung von 42,6 g (0,3 Mol) 3,4-Ethylendioxythiophen (EDT) in 70 ml Methylenchlorid 2,13 g Trifluoressigsäure in 90 ml Methylenchlorid bei 18°C zugegeben. Das Reaktionsgemisch wurde 48 h unter N₂-Atmosphäre bei 18°C gerührt. Danach wurde die Reaktion durch intensives Rühren mit 150 ml 5 %iger wässriger Na₂CO₃-Lösung gestoppt. Die organische Phase wurde abgetrennt, mit Wasser neutral gewaschen und am Rotationsverdampfer bei 20 mbar vom Lösungsmittel befreit. Der Rückstand (42,2 g) wurde an Silicagel säulenchromatographisch mit Methylenchlorid als Laufmittel aufgetrennt.
- Fraktion 1:: 21 g EDT
- Fraktion 2:: 11 g EDT-Dimer, identifiziert mittels ¹H-NMR in CDCl₃. Beige Kristalle,
- Schmp.:: 115-119 °C.
- Fraktion 3:: 1 g EDT-Trimer als Isomerengemisch, identifiziert mittels ¹H-NMR in CDCl₃. Beige Kristalle.

Das Produkt aus Fraktion 2 wird in den erfindungsgemäßen Beispielen 1 und 2 eingesetzt.

### Herstellung von Dimethyl-2,2',3,3',5,7-hexahydro-5,5'-bithieno[3,4-b] [1,4] dioxin (II-b) (Methyl-EDT-Dimer) durch BF₃-Katalyse

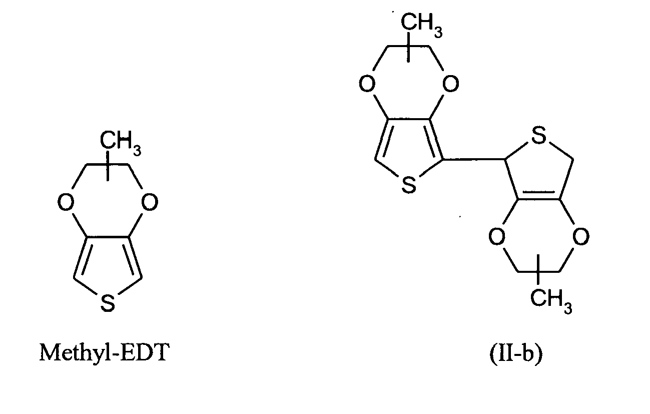

Wie in der allgemeinen Vorschrift beschrieben, wurden zu einer Lösung von 6,25 g (40 mMol) 2-Methyl-2,3-dihydrothieno[3,4-b][1,4]dioxin (Methyl-EDT) in 30 ml Methylenchlorid 0,24 ml Bortrifluorid-Etherat (entsprechend 269 mg = 1,89 mMol) in 40 ml Methylenchlorid bei 23°C zugegeben. Das Reaktionsgemisch wurde 7 h unter N₂-Atmosphäre bei 23°C gerührt. Danach wurde die Reaktion durch Zugabe von 20 ml Ethanol gestoppt. Die organische Phase wurde mit Wasser neutral gewaschen und am Rotationsverdampfer bei 20 mbar vom Lösungsmittel befreit. Der Rückstand wurde an Silicagel säulenchromatographisch mit Methylenchlorid als Laufmittel aufgetrennt. Ausbeute: 1,3 g Methyl-EDT-Dimer (II-b) (20,8 % d. Th.).

### Herstellung von [(Hydroxymethyl)-2,2',3,3',5,7-hexahydro-5,5'-bithieno[3,4-b][1,4]dioxinyl]methanol (II-c) (Hydroxymethyl-EDT-Dimer)

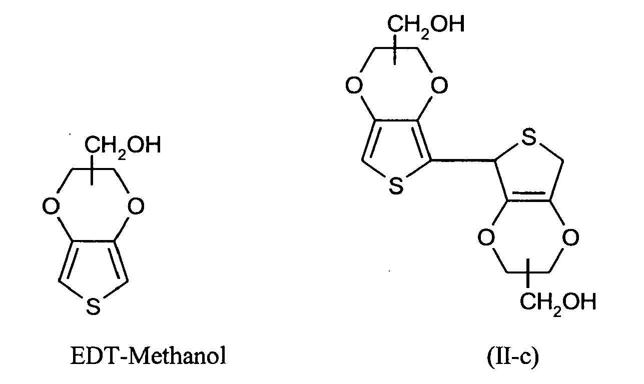

Wie in der allgemeinen Vorschrift beschrieben, wurden zu einer Lösung von 6,89 g (40 m Mol) 2,3-Dihydrothieno[3,4-b][1,4]dioxin-2-ylmethanol (Hydroxymethyl-EDT, "EDT-Methanol") in 90 ml Methylenchlorid 0,24 ml Bortrifluorid-Etherat (entsprechend 269 mg = 1,89 mmol) in 120 ml Methylenchlorid bei 0°C zugegeben. Das Reaktionsgemisch wurde 4 h unter N₂-Atmosphäre bei 0°C gerührt. Danach wurde die Reaktion durch Zugabe von einem Gemisch aus 60 ml Ethanol/120 ml Wasser gestoppt. Die organische Phase wurde abgetrennt und am Rotationsverdampfer bei 20 mbar vom Lösungsmittel befreit. Der Rückstand wurde an Silicagel säulenchromatographisch mit Methylenchlorid als Laufmittel aufgetrennt. Ausbeute 2,0 g Hydroxymethyl-EDT-Dimer (II-c) (29 % d. Th.)

### Beispiel 1: Herstellung von 2,2'-Di(3,4-ethylendioxythiophen) (I-a) (erfindungsgemäß)

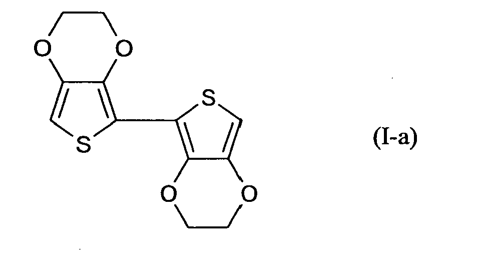

28,64 g (0,1 Mol) EDT-Dimer (II-a) wurden in 500 ml Xylol gelöst. Zu dieser Lösung wurden 24,59 g (0,1 Mol) Chloranil zugegeben. Das Gemisch wurde 10 h bei 140°C zum Rückfluss erhitzt. Nach Abkühlen wurden die ausgefallenen Kristalle abgesaugt und sowohl die Kristalle als auch die Mutterlauge weiter aufgearbeitet.

Die Mutterlauge wurde 4 mal mit je 50 ml 4 %iger Kalilauge gewaschen und zum Schluss mit Wasser neutral gewaschen. Die Lösung wurde eingedampft und im Hochvakuum getrocknet; man erhielt 15,8 g Rohprodukt als grüne Kristalle (Fraktion A).

Die abgesaugten Kristalle wurden mit 150 ml Xylol und 100 ml 4 %iger Kalilauge aufgeschlämmt und die unlöslichen Anteile (5,5 g) abfiltriert. Die Mutterlauge wurde wie die obige Mutterlauge weiter aufgearbeitet und ergab nach Eindampfen weitere 6,4 g Rohprodukt als grüne Kristalle (Fraktion B).

Die Rohprodukte (Fraktion A und B) wurden in 370 ml Methylenchlorid gelöst und mit 21 g Tonsil^{®} (Bleicherde vom Montmorillonit-Typ, Hersteller: Süd-Chemie, München) aufgeschlämmt. Nach Entfärbung der Lösung wurde das Tonsil^{®} abfiltriert und das Methylenchlorid am Rotationsverdampfer entfernt.

Es werden 18 g (63 % der Theorie) 2,2'-Di(3,4-ethylendioxythiophen) (I-a) als hellgelbe Kristalle isoliert. Schmp.: 204 - 205 °C.

¹H-NMR-Spektrum (400 MHz, CDCl₃, ppm): 4,24 (4H, m), 4,31 (4H, m), 6,27 (2H, s).

Aus den 5,5 g unlöslichen Anteilen wurden durch Aufschlämmen mit Tonsil^{®} in Methylenchlorid nach der gleichen Methodik weitere 1,5 g 2,2'-Di(3,4-ethylendioxythiophen) gewonnen. Gesamtausbeute: 69 % der Theorie.

### Beispiel 2: Herstellung von 2,2'-Di(3,4-ethylendioxythiophen) (I-a) mit 2,3-Dichlor-5,6-dicyan-p-benzochinon

8,53 g (30 mmol) EDT-Dimer (II-a) wurden in 150 ml Xylol gelöst. Zu dieser Lösung wurden 7,15 g (31,5 mmol) 2,3-Dichlor-5,6-dicyan-p-benzochinon zugegeben. Das Gemisch wurde 24 h bei 140 °C zum Rückfluss erhitzt. Nach Abkühlen wurde mit 4 %iger Kalilauge ausgeschüttelt, bis die Kalilauge farblos blieb. Zum Schluss wurde mit Wasser neutral gewaschen. Die Lösung wurde eingedampft und im Hochvakuum getrocknet; man erhielt 3,68 g (43,5 % d. Th.) Rohprodukt als graubraunen Rückstand. Dieses Rohprodukt wurde chromatographisch über eine Silicagel-Säule gereinigt, Laufmittel Methylenchlorid.

Es wurden 1,32 g (15,6 % der Theorie) reines 2,2'-Di(3,4-ethylendioxythiophen) (I-a) isoliert. Schmp.: 207 - 209 °C.

Das ¹H-NMR-Spektrum (400 MHz, CDCl₃) ist identisch mit dem in Beispiel 1 angegebenen.

### Beispiel 3: Herstellung von Dimethyl-2,2',3,3'-tetrahydro-5,5'-bithieno[3,4-b][1,4]dioxin (I-b)

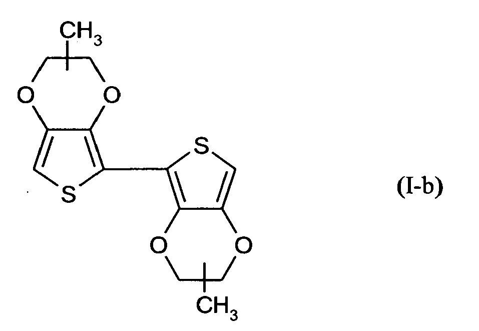

0,85 g (2,7 mmol) Methyl-EDT-Dimer (II-b) wurden in 50 ml Xylol gelöst. Zu dieser Lösung wurden 0,668 g (2,7 mmol) Chloranil zugegeben. Das Gemisch wurde 14 h bei 140°C zum Rückfluss erhitzt. Nach Abkühlen wurde mit Methylenchlorid verdünnt und mit 4%iger Kalilauge ausgeschüttelt, bis die Kalilauge farblos blieb. Zum Schluss wurde mit Wasser neutral gewaschen. Die Lösung wurde eingedampft und im Hochvakuum getrocknet; man erhielt 0,48 g (56,7 % d. Th.) rohes (I-b) als Rückstand. Das Produkt wurde säulenchromatographisch an Kieselgel, Laufmittel CH₂Cl₂, gereinigt. Reinausbeute 0,38 g (44,8 % d. Th.).

¹H-NMR-Spektrum (400 MHz, CDCl₃, ppm): 6,24, 6,235, 6,23 (3 s, zus. 2H); 4,39 (weiter aufgespaltenes q, ³*J* = 6,56 Hz) und 4,32 (weiter aufgespaltenes q, ³*J* = 6,56 Hz, beide q zus. 2H); 4,28 (dd, ²*J* = 11,60 Hz, ³*J* = 2,04 Hz) und 4,15 (dd, ²*J* = 11,60 . Hz, ³*J* = 2,04 Hz, beide dd zus. 2 H); 3,91 (dd, ²*J* = 11,60 Hz, ³*J* = 8,56 Hz) und 3,865 (dd, ²*J* = 11,60 Hz, ³*J* = 8,56 Hz, beide dd zus. 2H); 1,42 (d, ³*J* = 6,56 Hz) und 1,355 (d, ³*J* = 6,56 Hz, beide d zus. 6H). Das NMR-Spektrum legt das Vorliegen eines Produktgemisches aus folgenden Komponenten nahe:

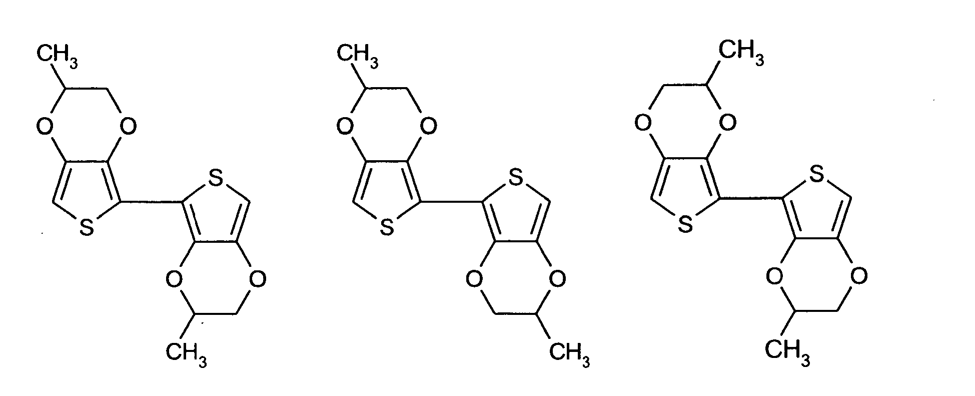

### Beispiel 4: Herstellung von [(Hydroxymethyl)-2,2',3,3'-tetrahydro-5,5'-bithie-no[3,4-b][1,4]dioxinyl]methanol (I-c)

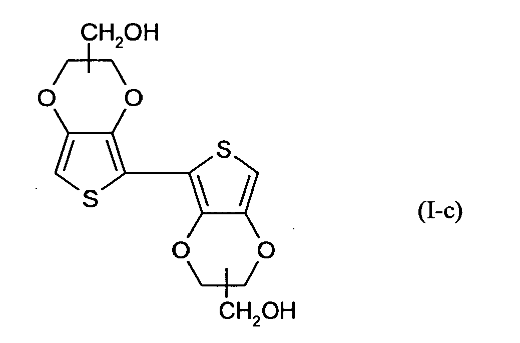

2,4 g (7 mmol) Hydroxymethyl-EDT-Dimer (II-c) wurden in 50 ml Xylol gelöst. Zu dieser Lösung wurden 1,713 g (7 mmol) Chloranil zugegeben. Das Gemisch wurde 24 h bei 140°C zum Rückfluss erhitzt. Nach Abkühlen wurde mit 4 %iger Kalilauge ausgeschüttelt, bis die Kalilauge farblos blieb. Zum Schluss wurde mit Wasser neutral gewaschen. Die Lösung wurde eingedampft und im Hochvakuum getrocknet; man erhielt 0,2 g (8,3 % d. Th.) (I-c) als Rückstand.

¹H-NMR-Spektrum (400 MHz, CDCl₃, ppm): 6,285; 6,275; 6,27; 6,26 (4 s, zus. 2H), 4,21 - 3,73 (mehrere m, zus. 6H). Das NMR-Spektrum legt das Vorliegen eines Produktgemisches aus folgenden Komponenten nahe:

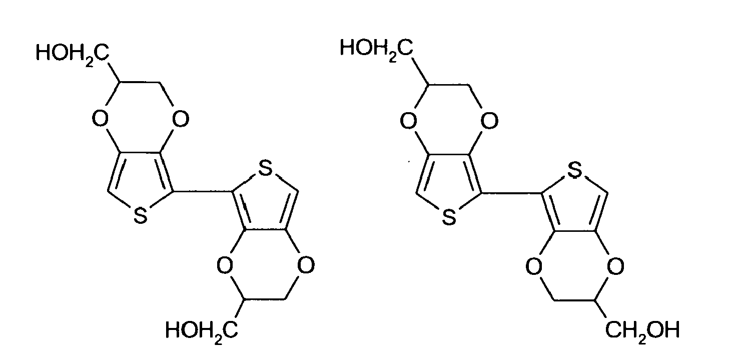

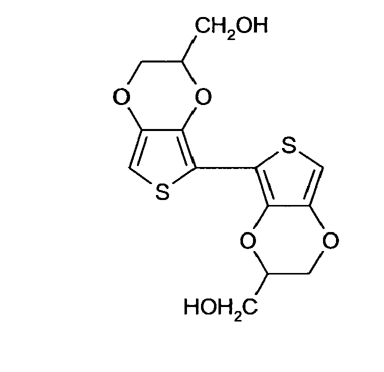

## Patentansprüche

1. Verfahren zur Herstellung von neutralen oder kationischen Verbindungen
der allgemeinen Formel (IV), 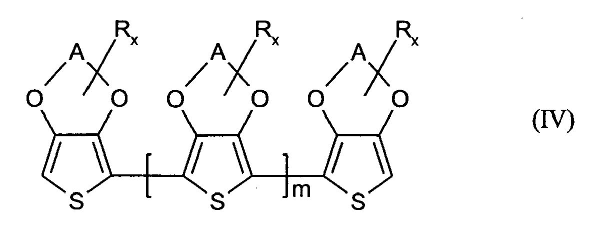 worin
R für einen oder mehrere, gleiche oder verschiedene lineare(n) oder verzweigte(n), gegebenenfalls substituierte(n) C₁-C₁₈-Alkylrest(e), gegebenenfalls substituierte(n) C₅-C₁₂-Cycloalkylrest(e), gegebenenfalls substituierte(n) C₆-C₁₄-Arylrest(e), gegebenenfalls substituierte(n) C₁-C₄-Hydroxyalkylrest(e) oder einen oder mehrere Hydroxylrest(e) steht,
x für eine ganze Zahl von 0 bis 8 steht,
m für eine ganze gerade Zahl von 2 bis 200 steht und
wobei die Verbindungen der allgemeinen Formel (IV) für den Fall, dass sie kationisch sind eine bis maximal m+2 positive Ladungen tragen,
**dadurch gekennzeichnet, dass** Verbindungen der allgemeinen Formel (I) 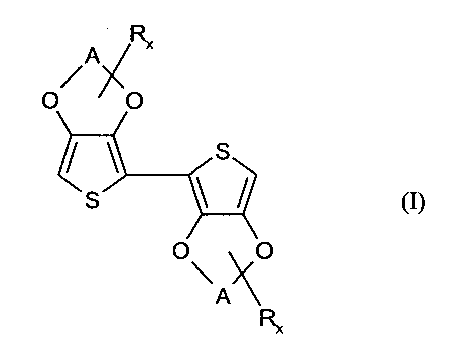 worin
A, R und x die vorstehend genannte Bedeutung haben,
chemisch oder elektrochemisch oxidativ polymerisiert werden.

2. Verfahren nach Anspruch 1, **dadurch gekennzeichnet, dass**
A für einen gegebenenfalls substituierten Ethylenrest steht,
R für einen oder mehrere, gleiche oder verschiedene lineare(n) oder verzweigte(n), gegebenenfalls substituierte(n) C₁-C₁₈-Alkylrest(e), gegebenenfalls substituierte(n) C₅-C₁₂-Cycloalkylrest(e), gegebenenfalls substituierte(n) C₆-C₁₄-Arylrest(e), gegebenenfalls substituierte(n) C₁-C₄-Hydroxyalkylrest(e) oder einen oder mehrere Hydroxylrest(e) steht,
x für eine ganze Zahl von 0 bis 4 steht.

3. Verfahren nach Anspruch 1 oder 2, **dadurch gekennzeichnet, dass**
A für einen gegebenenfalls substituierten Ethylenrest steht,
R für Methyl, Ethyl, n-Propyl, n-Butyl, n-Hexyl, n-Octyl, n-Decyl, n-Dodecyl, n-Tetradecyl oder Hydroxymethyl steht,
x für 0 oder 1 steht.

4. Verfahren nach einem der vorhergehenden Ansprüche, **dadurch gekennzeichnet, dass** die Verbindungen der allgemeinen Formel (I) chemisch oxidativ polymerisiert werden.

5. Verfahren nach Anspruch 4, **dadurch gekennzeichnet, dass** als chemisches Oxidationsmittel Eisen-III-Verbindungen eingesetzt werden.

6. Verfahren nach Anspruch 5, **dadurch gekennzeichnet, dass** die Eisen-III-Verbindung Eisen-III-tosylat ist.

7. Verfahren nach Anspruch 1, **dadurch gekennzeichnet, dass** die Verbindungen der allgemeinen Formel (IV) kationisch sind und zur Kompensation der positiven Ladung Polyanionen als Gegenionen enthalten.

8. Verfahren nach Anspruch 7, **dadurch gekennzeichnet, dass** die Polyanionen Polystyrolsulfonsäuren sind.

9. Verfahren nach Anspruch 7 oder 8, **dadurch gekennzeichnet, dass** das Molekulargewicht der Polyanionen 1.000 bis 2.000.000 beträgt.

10. Verfahren nach Anspruch 7 oder 8, **dadurch gekennzeichnet, dass** das Molekulargewicht der Polyanionen 2.000 bis 500.000 beträgt.
